Europäisches Patentamt

European Patent Office

Office européen des brevets

(11) Veroffentlichungsnummer: **0 133 502**

**A2**

(12) **EUROPÄISCHE PATENTANMELDUNG**

(21) Anmeldenummer: **84108612.7**

(51) Int. Cl.⁴: **G 01 N 27/16**

(22) Anmeldetag: **20.07.84**

(30) Priorität: **05.08.83 DE 3328385**

(43) Veröffentlichungstag der Anmeldung:
**27.02.85 Patentblatt 85/9**

(84) Benannte Vertragsstaaten:
**CH DE FR IT LI NL SE**

(71) Anmelder: **KRAFTWERK UNION
AKTIENGESELLSCHAFT
Wiesenstrasse 35
D-4330 Mülheim (Ruhr)(DE)**

(72) Erfinder: **Odar, Suat, Dr. Dipl.-Ing.
Killingerstrasse 35A
D-8520 Erlangen(DE)**

(72) Erfinder: **Schaller, Helmut
Rainbügl 13 1/2
D-8430 Neumarkt(DE)**

(74) Vertreter: **Mehl, Ernst, Dipl.-Ing. et al,
Postfach 22 01 76
D-8000 München 22(DE)**

(54) **Sonde zur Feststellung von brennbaren Gasen.**

(57) Bei einer Sonde mit einem Diffusionsfernmeßkopf (1) zur Feststellung von brennbaren Gasen mit einem Gehäuse (2), das mindestens einen Detektorwendel (4, 5) enthält, der mit dem Gas beaufschlagt wird, ist an dem Gehäuse (2) eine Heizeinrichtung (16) vorgesehen, die das Gehäuse (2) auf einer Temperatur von mehr als 100°C hält. Die Heizeinrichtung (16) wird vorzugsweise über einen Temperaturfühler (19) geregelt, so daß das Gehäuse (2) eine konstante Temperatur im Bereich von 105°C bis 120°C mit Abweichungen von weniger als 5°C aufweist. Die Sonde kommt insbesondere zur Feststellung von Wasserstoff in Kernkraftwerken in Frage.

FIG 1

KRAFTWERK UNION AKTIENGESELLSCHAFT   Unser Zeichen

VPA 83 P 6043 E

Sonde zur Feststellung von brennbaren Gasen

Die Erfindung betrifft eine Sonde zur Feststellung von brennbaren Gasen mit einem Gehäuse, das mindestens einen Detektorwendel enthält, der mit dem Gas beaufschlagt wird.

Eine Sonde der vorgenannten Art, die mit einem Diffusionsfernmeßkopf zur Feststellung von Wasserstoff in einem Kernkraftwerk dient, ist in der DE-OS 30 46 560 beschrieben. Sie besitzt einen Detektorwendel und einen Kompensationswendel, die in Brückenschaltung angeordnet sind, damit die Einwirkungen des Wasserstoffs beim Oxidieren möglichst empfindlich erfaßt werden können. Die bekannte Sonde ist mit Hilfe von Siliconkautschuk und besonders strahlungsresistenten Isolierstoff für den sogenannten GAU-Fall geeignet ausgebildet, bei dem trotz hoher Strahlungsintensität, hoher Temperaturen und großer Feuchtigkeit (Dampf) das sichere Arbeiten gewährleistet sein muß.

Ziel der Erfindung ist eine weitere Verbesserung der vorgenannten Sonde, denn bei Langzeiterprobungen hat sich gezeigt, daß die Meßgenauigkeit solcher Sonden zunächst unerklärlichen Schwankungen unterworfen war.

Gemäß der Erfindung ist vorgesehen, daß an dem Gehäuse eine Heizeinrichtung vorgesehen ist, die das Gehäuse auf einer Temperatur von mehr als 100°C hält.

Bei der neuen Sonde wird durch die Heizeinrichtung ver-

Sm 2 Hgr / 02.08.1983

mieden, daß die durch den Meßvorgang, üblicherweise eine Verbrennung, beanspruchten Detektor- und gegebenenfalls Kompensationswendel in ihrer Wirkungsweise beeinträchtigt werden. Zum Beispiel ist für Wasserstoff als festzustellendes Gas mit der hohen Temperatur sichergestellt, daß das beim Meßvorgang entstehende Wasser sofort dampfförmig ausgetrieben wird und sich nicht etwa als Kondensat an den Wendeln absetzt.

Die Gehäusetemperatur liegt vorzugsweise im Bereich von 105 bis 120°C. In diesem Bereich ist die genannte Verdampfung sichergestellt, ohne daß durch überhöhte Temperaturen der mechanische Aufbau der Sonde oder eine organische Isolierung elektrischer Teile oder zum Kleben dienende Kunststoffe gefährdet wären. Man kann aber im Prinzip auch höhere Temperaturen wählen, insbesondere dann, wenn im GAU-Fall mit hohem Dampfgehalt in der Umgebung der Sonde zu rechnen ist.

Bei einer bevorzugten Ausführungsform der Erfindung ist die Heizeinrichtung über einen Temperaturfühler geregelt, so daß das Gehäuse eine konstante Temperatur mit Abweichungen von weniger als 5°C aufweist. Die konstante Temperatur sorgt für gleichbleibende Zustände beim Meßvorgang. Sie kann insbesondere für die Feststellung von Wasserstoff mit noch geringeren Abweichungen eingeregelt werden, die dann vorzugsweise höchstens 2°C betragen.

Die Erfindung kann mit verschiedenen Heizeinrichtungen und unterschiedlichen bekannten Temperaturfühlern verwirklicht werden. Besonders bewährt hat sich eine Sonde, bei der der Temperaturfühler ein Widerstandsthermometer ist. Als Temperaturfühler kann aber auch ein Bimetallschalter eingesetzt werden, wie er üblicherweise zur Regelung kleiner Heizleistungen, zum
Beispiel in einem Heizkissen, vorgesehen ist.

Die Heizeinrichtung ist vorzugsweise eine Widerstandsheizung mit keramischen Tragteilen. Im Prinzip sind
aber auch andere Heizeinrichtungen denkbar, zum Beispiel solche, die das den Detektorwendel einschließende Gehäuse direkt durch Wirbelstrom erwärmen, wenn
es sich um ein Metallgehäuse handelt. Auch Hochfrequenzerwärmungen eines den Detektorwendel tragenden
Isolierstoffes sind vorstellbar.

In jedem Fall ist es günstig, wenn die Heizeinrichtung
mit dem Temperaturfühler zu einem Bauelement zusammengesetzt ist, das mit dem Gehäuse wärmeleitend verbunden ist. Eine derartige Bauweise als Bauelement gestattet die Vorfertigung bzw. die Nachrüstung fertiger Sonden. Dies gilt besonders für den Fall, daß das
Bauelement einen Metallblock umfaßt, der mit dem Gehäuse zusammengespannt ist. Zum Zusammenspannen kann
eine Verschraubung dienen, wie später anhand des Ausführungsbeispiels näher erläutert wird. Das Bauelement kann aber auch in das Gehäuse einer Sonde unmittelbar eingesetzt sein.

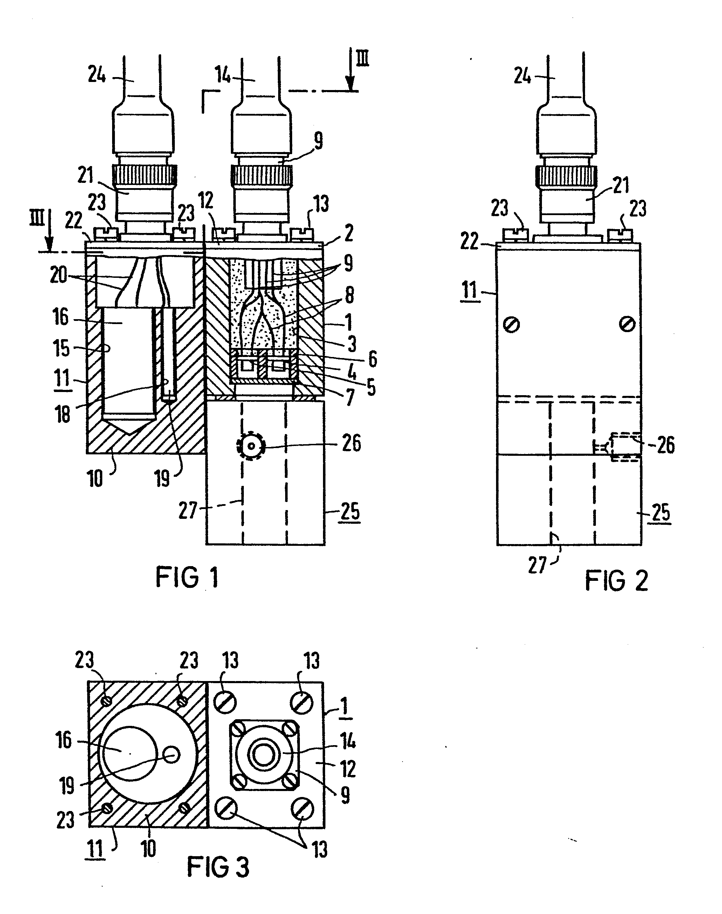

Weitere Einzelheiten der Erfindung ergeben sich aus der
folgenden Beschreibung eines Ausführungsbeispiels, das
in der Zeichnung dargestellt ist. Dabei zeigt die Fig. 1
eine Prüfanordnung für eine Sonde nach der Erfindung
in einer Seitenansicht, zum Teil im Schnitt. Die Fig. 2
und 3 sind zwei weitere jeweils um 90° gedrehte Darstellungen.

Mit 1 ist ein Diffusionsfernmeßkopf bezeichnet, der
so aufgebaut ist wie die in der DE-OS 30 46 560 erläuterte Sonde. Er umfaßt also als wesentlichen Teil einen Gehäusegrundkörper 2 aus Edelstahl. Der Gehäusegrundkörper 2 mit seiner im wesentlichen würfelförmigen Gestalt umschließt einen durchgehend zylindrischen Hohlraum 3. In diesem sitzen zwei benachbart angeordnete wendelförmige Drahtelektroden 4, 5 (Detektor- und Kompensationswendel) in zylindrischen Ausnehmungen einer in den Hohlraum 3 eingeschobenen, außen zylindrischen Scheibe 6 aus Edelstahl. Die Detektorwendel sind durch eine Sintermetallplatte 7 abgeschirmt, die aufgrund ihrer Porosität den Durchtritt der zu überwachenden Gasatmosphäre des Sicherheitsbehälters eines Kernkraftwerkes gestattet.

Die Wendel 4, 5 sind mit Anschlußdrähten 8 zu einem Stecker 9 geführt, der auf einer Verschlußplatte 12 angebracht ist, die mit Schrauben 13 auf den Grundkörper 2 aufgepreßt wird. Der Stecker 9 führt zu einem Anschlußkabel 14.

An dem Grundkörper 2 des Meßkopfes 1 ist der Metallblock 10 eines Bauelements 11 angepreßt, der mit Schrauben befestigt wird und ebenfalls aus Edelstahl besteht. Der im Querschnitt dem Grundkörper 2 gleiche Metallblock 10 hat innen im Anschluß an eine zentrale zylindrische Ausnehmung einen vertikal verlaufenden Kanal 15 für eine nicht weiter dargestellte Heizpatrone 16 für zum Beispiel 50 W mit einem Keramikkörper und einem Widerstandsheizdraht. Der Durchmesser des Kanals 15 ist etwa halb so groß wie die Dicke des Querschnitts des Metallblockes 10.

Parallel zu dem Kanal 15 verläuft im Metallblock 10 ein Meßkanal 18 mit einem wesentlich, zum Beispiel um 2/3, kleineren Durchmesser. In diesem Meßkanal ist ein nicht weiter dargestelltes Widerstandsthermometer 19 untergebracht, so daß es dem Grundkörper 2 der Sonde dicht benachbart ist. Damit kann die Temperatur des Grundkörpers 2 insbesondere im Bereich der Wendel 4, 5 genau auf einen für die Heizeinrichtung vorgegebenen Wert gehalten und gebracht werden. Die Anschlußleitungen 20 der Heizeinrichtung 16 und des als Temperaturfühler dienenden Widerstandsthermometers 19 führen zu einem Stecker 21, der auf einer Verschlußplatte 22 mit Schrauben 23 befestigt ist. Der Stecker 21 bildet den Übergang zu einem Anschlußkabel 24.

Beim Ausführungsbeispiel ist der Diffusionsfernmeßkopf 1 der Sonde mit einem Prüfkopf 25 versehen. Der Prüfkopf 25 besitzt einen Prüfgasanschluß 26 für einen Gaskanal 27. Damit wird zur Prüfung der Sonde über einen bestimmten Zeitraum von zum Beispiel Stunden oder sogar Tagen eine Wasserstoffatmosphäre von bis zu 8% an den Diffusionsmeßkopf 1 gebracht, die von diesem mit einer Genauigkeit von ± 0,2 bis ± 0,5 Vol.-% festgestellt und gemeldet werden muß. Im Normalfall, d.h. bei einem Einsatz in einem Kernkraftwerk, ist der Prüfkopf 25 für fernbetätigte Prüfungen vorhanden, mit denen für unzugängliche Sonden die Funktionsfähigkeit nachgewiesen wird.

Trotz der hohen Gaskonzentration arbeitet die neue Sonde einwandfrei. Auch im Dauerbetrieb kommt es nicht zu unzulässigen Schwankungen der Meßgenauigkeit. Dies wird wesentlich dadurch mitbestimmt, daß das in gut wärmeleitendem Kontakt mit dem Grundkörper 2 stehende Bauelement 11 für eine konstante Temperatur von 110°C mit einer Abweichung von nur plus/minus 1,5°C sorgt. Damit wird ein Auskondensieren des durch den Meßvorgang entstehenden Wasserdampfes vermieden und der Dampf aus der Meßkammer, d.h. aus dem Bereich der Wendel 4, 5 des Diffusionsmeßkopfes 1 ausgetrieben.

Das Bauelement 11 ist wie die Sonde GAU-fest gestaltet. Dies bedeutet, daß der zum Befestigen der Heizpatrone 16 und des Temperaturfühlers 19 dienende Kunststoffkleber ebenso wie der Siliconkautschuk strahlungsresistent ist, der den Hohlraum für die Anschlußdrähte 20 zum Stecker 21 füllt. Ebenso ist das Kabel 24 strahlungsresistent isoliert, zum Beispiel mit einer mineralischen Isolierung.

Als alternative Lösung für die Beheizung der Sonde könnte die Heizpatrone auch in den Grundkörper 2 integriert werden. Eine dafür besonders geeignete Form besteht in einer konzentrischen Anordnung, bei der in der Mitte die Detektor- und Kompensationswendel 4,5 liegen. Sie werden von einem Kanal für den Temperaturfühler umschlossen. Um diesen ist ein weiterer Kanal für die Heizeinrichtung gelegt.

8 Patentansprüche

3 Figuren

0133502

Patentansprüche

1. Sonde zur Feststellung von brennbaren Gasen, insbesondere Wasserstoff, in Kernkraftwerken, mit einem Gehäuse, das mindestens einen Detektorwendel enthält, der mit dem Gas beaufschlagt wird, d a d u r c h  g e - k e n n z e i c h n e t, daß an dem Gehäuse (2) eine Heizeinrichtung (16) vorgesehen ist, die das Gehäuse (2) auf einer Temperatur von mehr als 100°C hält.

2. Sonde zur Feststellung von brennbaren Gasen nach Patentanspruch 1, d a d u r c h  g e k e n n z e i c h - n e t, daß die Gehäusetemperatur 105 bis 120°C beträgt.

3. Sonde zur Feststellung von brennbaren Gasen nach Patentanspruch 1 oder 2, d a d u r c h  g e k e n n - z e i c h n e t, daß die Heizeinrichtung (16) über einen Temperaturfühler (19) geregelt wird, so daß das Gehäuse (2) eine konstante Temperatur mit Abweichungen von weniger als 5°C aufweist.

4. Sonde zur Feststellung von brennbaren Gasen nach Patentanspruch 3, d a d u r c h  g e k e n n z e i c h - n e t, daß der Temperaturfühler ein Widerstandsthermometer (19) ist.

5. Sonde zur Feststellung von brennbaren Gasen nach Patentanspruch 3, d a d u r c h  g e k e n n z e i c h - n e t, daß der Temperaturfühler ein Bimetallschalter ist.

6. Sonde zur Feststellung von brennbaren Gasen nach Patentanspruch 3, 4 oder 5, d a d u r c h  g e k e n n - z e i c h n e t, daß der Temperaturfühler (19) zwischen Heizeinrichtung (16) und  Detektorwendel (4, 5) liegt.

7. Sonde zur Feststellung von brennbaren Gasen nach Patentanspruch 3 bis 6, d a d u r c h   g e k e n n - z e i c h n e t, daß die Heizeinrichtung (16) mit dem Temperaturfühler (19) zu einem Bauelement (11) zusammengesetzt ist, das mit dem Gehäuse (2) wärmeleitend verbunden ist.

8. Sonde zur Feststellung von brennbaren Gasen nach Patentanspruch 7, d a d u r c h   g e k e n n z e i c h - n e t, daß das Bauelement (11) einen Metallblock (10) umfaßt, der mit dem Gehäuse (2) zusammengespannt ist.

0133502

83 P 6043

FIG 1

FIG 2

FIG 3